# EUROPEAN PATENT APPLICATION

(11) **EP 3 965 037 A1**
(43) Date of publication of application: **09.03.2022**
(21) Application number: 21204463.0
(22) Date of filing: 07.09.2009
(51) Int. Cl.: G06Q 10/10, H04M 3/42, G06F 21/62

(54) **MONITORING SYSTEM**

(30) Priority: 05.09.2008 FI 20085836; 30.04.2009 FI 20095489
(62) Divisional of application: 09811159.4
(71) Applicant: Suomen Terveystalo Oy, 00100 Helsinki (FI)
(72) Inventor: Parvinen, Arvi, 00170 Helsinki (FI); Parvinen, Lasse, 02400 Kirkkonummi (FI)
(74) Representative: Boco IP Oy Ab

(57) **Abstract**

The invention embodies a monitoring system (131) that is connected to one or more databases (101, 102, 103, 104) including data entries that represent status for said individual in the respective database. The monitoring system uses data entries of a group of individuals resulting from the search to form, for each individual in the group of individuals, a first system status representing status for said individual in the monitoring system. The first system statuses are exposed to a statistical analysis to form a second system status representing status for the group of individuals in the monitoring system.

## Description

### Field of the invention

The invention relates to a monitoring system as defined in the preamble of an independent monitoring system claim.

The invention also relates to a monitoring system element as defined in the preamble of an independent claim thereof.

The invention also relates to a monitoring method as defined in the preamble of an independent claim thereof.

The invention also relates to a monitoring program code as defined in the preamble of an independent claim thereof.

The invention also relates to an anonymizing interface for the monitoring system as defined in the preamble of an independent claim thereof.

The invention also relates to method of collecting information from information systems, especially collecting performed via the anonymizing interface as defined in the preamble of an independent claim on method of collecting information.

The invention also relates to a method of transforming the collected information to statistical information as defined in the preamble of an independent claim on method of transforming the collected information to statistical information.

The invention also relates to a business method as defined in the preamble of an independent claim thereof.

The invention also relates to an arrangement for collecting and/or storing of statistical information in statistical form as defined in the preamble of an independent claim on the arrangement.

The invention also relates to a monitoring system as defined in the preamble of an independent monitoring system claim.

### Back ground

Collection of information as such from data networks can be implemented via the network interfaces. There are many data mining algorithms as such available. In data mining as such, large amount of different kind of data can be searched, and the found data items categorized as such according to the search criterion. The mined data can be reorganized to form structures that are categorized according to the relevance of a criterion for the re-organization where necessary. In data mining, also meta-data as such can be used to describe the actual data and so making easier to avoid non-relevant data. Data as such may be available as such in many ways from the numerous sources, that are connected to information network, in a directly accessible way or via supervised control through at least some secrecy.

Especially such information that concern on individual persons for example is not publically accessible, although may be in technical sense available as such. The confidentiality and the misuse risk -real or not- restrict use of such information, except in cases where the individual itself voluntarily allows the public access to the data. Thus, many interesting pieces of information are protected because of the individual privacy protection. Such a good aspect for an individual also limits the use and combining of such information for the goals aiming for the best of the individual. Information privacy may be defined in a specific law, and/or in an internal code of a community. Information that concern or reveals the identity of an individual are often protect under such law or code, so that the information is not accessible without the permission of the individual.

It is well grounded to make such information secret, in most cases. Thus, possible errors in the databases or in their connections do not cause harmful status to the individual. In such a way also erroneous information can be avoided to escape into publicity and so harming an individual. It is possible that combining information by machine may produce such information that may be harmful or at least have an intimate character, so preferably maintained with a status non-public. This may be the case, although the combination of the information were true as such.

In some countries the privacy information law (PIL) is used to define a norm to the privacy of an individual. The patient data bases on the physical and/or mental state of the patients or on other comparative data is not allowed to be given to anybody without the consent of the individual's own will. This concerns also cases in which the individual identity can be deduced from the information. In a working community, the local PIL prevents the access to the workers' health data, even for statistical analysis as such, which is problematic to service providers specializing on the outsourced services on well-ness of the employees. As the information having non-accessible status, the internal service providers do not have any need to give the information outside, but on the contrary, to hide the information as strictly as possible, on the employee health status. On the other hand, an individual should be able to trust on the identity maintained in secrecy, even then when information concerning the individual him-/herself is being processed. For the purpose, a limited ensemble of operators may thus gain rights to operate with the data in a restricted environment.

An other example of data accessible to a limited ensemble on interested individuals can be demonstrated via a wireless village community or via open mobile alliance OMA specifications, that are related to the presence as such in the mobile communication.

It is known that in a presence technique, a user client publishes the user's presence state to indicate its current communication status. The published state is used as an indicator to the others that wish to contact said user that so shows his availability and willingness to communicate. Common presence states comprise indicators to show that the current person is "free for chat", "busy", "home", "lunch" etc. Such states exist in many variations across different modern instant messaging clients. The standards available support a rich choice of additional presence attributes that can be used for presence information, such as user's mood, location, or even free text status.

However, there is a lot of information potentially available, but not accessible on individual persons. Such information belong information that is for instance shielded by the personal immunity demand, especially for such information that may be considered annoying or uncomfortable to the individual in question, sicknesses and weaknesses etc that are not considered pleasant for the individual itself to be associated with. For statistical analysis the information of individuals thus keep in secrecy and is not available, even for the actions and aims that would improve the wellness of the individual.

Furthermore, for the presence techniques as such in the known form, the individuals themselves put the information available and thus the system would show only convenient data for the individual or neutral data. Thus the presence has also a consequential disadvantage as such that the freedom of individuals lead to prejudiced data with colorful non-standard and consequently non-classifiable data.

### Objective of the invention

It is an object to solve the aforementioned problems, or at least mitigate the problems. The object is achieved with the embodiments of the invention. The object of the invention is to implement a monitoring system for monitoring observables that are comprised by ensemble of individual observables that are made anonymous for the monitoring of the individual observables as an ensemble so that the monitoring is based on an allotment of the information available for the individual observables. It is thus an objective of the invention to combine data-mining and a modified presence technology as such together via an interface for a monitoring system to be used in monitoring of observables. For the monitoring system implementation, another related object of the invention is to provide the monitoring system with an anonymizer, an anonymizing interface, to make observables anonymous in accordance of information protection criteria. In the light of the mentioned interrelated objects, it is a related further object to provide the user for the use of such information on the observables that can be used in controlling of various management systems of the user via the data, i.e. as control data.

In the following observable is considered as an individual that has certain data entries in a data base so forming a status of said observable. The data entries form discretion or an allotment of the status for said observable. The certain combination of the entries is considered to form the status in the data base.

When a data mining event is addressed to an observable having a status in the data base, the data mining of a monitoring system uses a certain ensemble of the entries to form a system status for the observable in the monitoring system that points the data mining act to the data base in question. Thus, the allotment in the monitoring system for the status of an observable can be different than the allotment in the original data base, but is not necessarily. An interface is used for the conversion from a first allotment to another allotment.. As the data entries made in the data base can be preferably made in a standard code, the allotment scaling can be made purpose specifically in the interface. Anonymizing interface may comprise these functionalities according to the respective embodiments as integrated into the anonymizer interface. In addition, any interfacing functionality of an embodiment or functionality of various means can be in suitable part integrated into the anonymizing interface, i.e. the interface that makes data anonymous when used on the data as embodied in embodiments of the invention.

A dynamic observable is built in the monitoring system from the observables and their status. As the number of the observables that are used for the constitution of particular dynamic observable may vary as well as the system status of the dynamic observable in the monitoring system, the observable is considered as dynamic. The dynamic observable can also have system status that is formed according to the status of the observables in a certain predetermined way. The status of observables may be transformed from one discretion to another, that in the monitoring system, by an interface of the monitoring system.

The monitoring system comprises also in one embodiment presentation means arranged to present a dynamic observable, and/or the system status of it. According to an embodiment of the invention the observables are shown that constitute the dynamic observable with the relevant system status. According to an embodiment of the invention allotment items of the allotment of system status the visibility thereof is restricted to certain items and/or their value according to the user's control, on line or in suitable part along a predetermined pattern.

### Short description of the invention

Monitoring system according to the invention is characterized in that what has been defined in the characterizing part of an independent monitoring system claim.

Further preferred embodiments relating to the monitoring system are shown as examples in the claims that dependent on the independent monitoring system claim.

Monitoring system element according to the invention is characterized in that what has been defined in the characterizing part of an independent system element claim.

Further preferred embodiments relating to the monitoring system element are shown as examples in the claims that dependent on the independent monitoring system element claim

Monitoring method according to the invention is characterized in that what has been defined in the characterizing part of an independent claim on monitoring method.

Further preferred embodiments relating to the monitoring method are shown as examples in the claims that dependent on the independent monitoring method claim.

Computer code according to the invention is characterized in that what has been defined in the characterizing part of an independent claim on computer code.

Further preferred embodiments relating to the monitoring computer code are shown as examples in the claims that dependent on the independent computer code claim.

An anonymizing interface for the monitoring system according to the invention is characterized in that what has been defined in the characterizing part of an independent claim on the interface.

Further preferred embodiments relating to the anonymizing interface are shown as examples in the claims that dependent on the independent anonymizing interface claim.

A method to convert information to statistical information according to the invention is characterized in that what has been said in the characterizing part of an independent claim directed to the method to convert information to statistical information.

Further preferred embodiments relating to the method are shown as examples in the claims that dependent on the independent method claim.

A business-method according to the invention is characterized in that what has been said in the characterizing part of an independent business-method claim,

Further preferred embodiments relating to the business-method are shown as examples in the claims that dependent on the independent business-method claim.

An arrangement for collecting and/or storing information is characterized in that what has been said in the characterizing part of an independent claim directed to the arrangement for collecting and/or storing information.

Further preferred embodiments relating to the arrangement for collecting and/or storing information are shown as examples in the claims that dependent on the independent arrangement claim for collecting and/or storing information.

In the following observable is considered as an individual that has certain data entries in a data base so forming a status of said observable. The data entries form discretion or an allotment of the status for said observable. The certain combination of the entries is considered to form the status in the data base.

When a data mining event is addressed to an observable having a status in the data base, the data mining of a monitoring system uses a certain ensemble of the entries to form a system status for the observable in the monitoring system that points the data mining act to the data base in question. Thus, the allotment in the monitoring system for the status of an observable can be different than the allotment in the original data base, but is not necessarily. An interface is used for the conversion from a first allotment to another allotment. As the data entries made in the data base can be preferably made in a standard code, the allotment scaling can be made purpose specifically in the interface.

A dynamic observable is built in the monitoring system from the observables and their status. As the number of the observables that are used for the constitution of particular dynamic observable may vary as well as the system status of the dynamic observable in the monitoring system, the observable is considered as dynamic. The dynamic observable can also have system status that is formed according to the status of the observables in a certain predetermined way. The status of observables may be transformed from one discretion to another, that in the monitoring system, by an interface of the monitoring system.

The monitoring system comprises also in one embodiment presentation means arranged to present a dynamic observable, and/or the system status of it. According to an embodiment of the invention the observables are shown that constitute the dynamic observable with the relevant system status. According to an embodiment of the invention allotment items of the allotment of system status the visibility thereof is restricted to certain items and/or their value according to the user's control, on line or in suitable part along a predetermined pattern.

In the following, a factor analysis as such refers to a normal factor analysis as known from the mathematics. However, additionally it refers in suitable part also to data mining and use algorithms that are used to search and combined data to information. Data as such can be classified in many ways for data-mining and/or the consequential purposes of it.

In an embodiment of the invention, the interface there between the data mining algorithm and the presence server to show the information on the ensemble of observable individuals can comprise factor analysis means arranged to be operable in combination in suitable part with classification means based on Naive Bayes classifier, k-nearest neighbor algorithm, another classification tree algorithm and/or a combination thereof. Also a Support vector machine or a variant thereof can be used in suitable part, alone or with a combination of classification means or as a variant thereof as adapted to the data mining algorithm as such to be used as majority classifier. In an embodiment, also logistic regression can be used in the interface. Implementation of the interface there between the data mining algorithm in the data mining means can comprise features of uplift modeling. Time series and the history data scoring can be used in the meta data formation in the in the interface boundaries. Anonymizing interface device may comprise these functionalities according to the respective embodiments as integrated in it as arranged for the operations between systems.

According to an embodiment, the data mining algorithm, the meta-data means arranged to handle metadata as well as the classification algorithm as such are known as such as well as the implementation of the presence server, but the combination that configures the just mentioned together by the interface is implemented in a new way.

According to an embodiment of the invention the observable individuals are made anonymous so that their data items in a data base comprises the presence data of the individuals, to be used in a data mining and/or factor analysis of the monitoring system.

According to an embodiment of the invention, dynamical observables are constituted dynamically from individual observables and such dynamical observables are given a system status in the allotment of the system status. According to an embodiment of the invention dynamical observables are used as meta data for data analysis and/or factor analysis.

According to an embodiment of the invention the interface of the monitoring system comprises means to emulate data bases as data bases of a presence server, so that the observables in the data base corresponds the presence users of a presence system, and the data items of the observables correspond the presence status of the users of presence system.

According to an embodiment of the invention the anonymizing interface can comprise control means arranged to influence on the interface operations, data base mountings/unmountings, search flow, the data mining algorithm selection for the data mining means, the filtration selection of the data and/or meta data, the access point and the user interface appearance, and/or to rights to access the monitoring system as well as to accesses to the data bases.

According to an embodiment the monitoring system comprises means for mounting/unmounting system elements, such as for example data bases.

According to an embodiment of the invention system elements can be parts of a same data base, but are not necessary restricted only thereto.

According to an embodiment of the invention a user can use the monitoring system to evaluate and compare service providers in the extent available via the access to the various data bases open or mountable to the monitoring system in use of the user. This is important also when a company for instance seeks a suitable wellness service provider or a personal care business to take care of the company's personal's welfare.

According to an embodiment of the invention the values of the data entries in the allotment of the system status are converted to points. For text values or for logical values a fixed amount of points can be used, according to a predetermined rate. The points can be given according to the probabilities of fulfilled value for an observable in question, or in an inverse manner in one embodiment variant. According to an embodiment the points, or scores, can be also negative, i.e. if the item were considered to influence negatively with that particular status for the wholeness. However, the scoring means in the monitoring system should keep book about the discretion and should carry information on the points and the corresponding data slot in the discretion, but so that the personal information is not revealed to any monitoring system parts in explicit, even if the observables were recognizable from data bases. In an embodiment the anonymizing interface may comprise means for these functionalities according to the respective embodiments as integrated in it, but is not limited only there to.

### List of figures

In the following the invention will described in more detail by referring to the following figures (FIG), which illustrate examples on preferred embodiments of the invention, as well as the examples shown later, as follows:
- FIG 1.: illustrates environment for embodiments,
- FIG 2: illustrates an embodiment according to the invention,
- FIG 3: illustrates an embodiment of the invention,
- FIG 4: illustrates a system status for an observable,
- FIG 5: illustrates supervisor's relations according to an embodiment of the invention,
- FIG 6 to FIG 24: illustrate implementation use and/or integration of embodiments in a connection to a SIRIUS system, for example,
- FIG 25: illustrate an example according to an embodiment of the invention,
- FIG 26: illustrate an example according to an embodiment of the invention, and
- FIG 27: illustrates an example according to an embodiment of the invention used for a business method.

Preferred embodiments are also indicated in the examples, as well as in the dependent claims. Similar reference numbers may be used in different FIGs, but they may be not necessarily exactly the same from figure to another, but rather, a skilled man in the art recognizes potential differences when read and understood the context.

### Detailed description of the invention

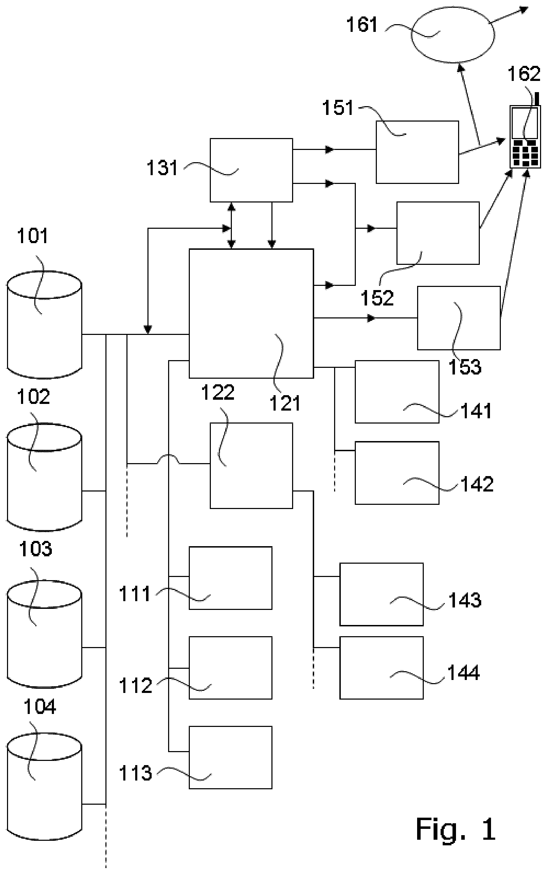

The FIG 1 illustrates an example on a scenario, where a company 121 as a member in a concern that comprises also the company 122, has bought services from service providers 141, 142, 143, 144, which may be service and/or company specific. Similar way the company 121 has bought insurance from an insurance company 111, medical services form a service provider 112, human resources from the service provider 113 or a personal rental firm, and other services from various service providers (not shown), for example. The company 121 has in its use also the data base 101 from the insurance company for the personal related information of the company. The company may have also other databases available, continuously mounted to its use or temporarily mounted/unmounted. The company has also access to the database on sickness funds 102 and data base of a retirement institution 103 used by the company 121 and/or the concern, as well as to data base of an injury register 104. The number of data bases and the detailed field as such are not limited only to the illustrative example above.

In the illustrative scenario, a monitoring system 131 according to an embodiment of the invention is connected to the company 121. The company has authorized an executive, or another person to operate as a supervisor to use the monitoring system. The arrows between monitoring system 131 and the data base branch illustrate communication facility including also searches in the name of the company 121 from the data bases. The company 121 has also an own server 153 available for informing legitimate clients 162 wirelessly on the company's events/occasions. In the scenario there is also shown another server 152, that can be similar as the server 153, but is not necessary exactly the same. The server has an access point to the legitimate users 162 wirelessly, but the server 152 can get input from the monitoring system 131 according to an embodiment of the invention, in addition to the input from the company 121. The server 151 is totally controlled by the monitoring system, for giving information on the system status of the observables detected and/or evaluated by the monitoring system. The illustration demonstrates access to this system status server 151 via internet 161 or via mobile access 162 for the legitimate users.

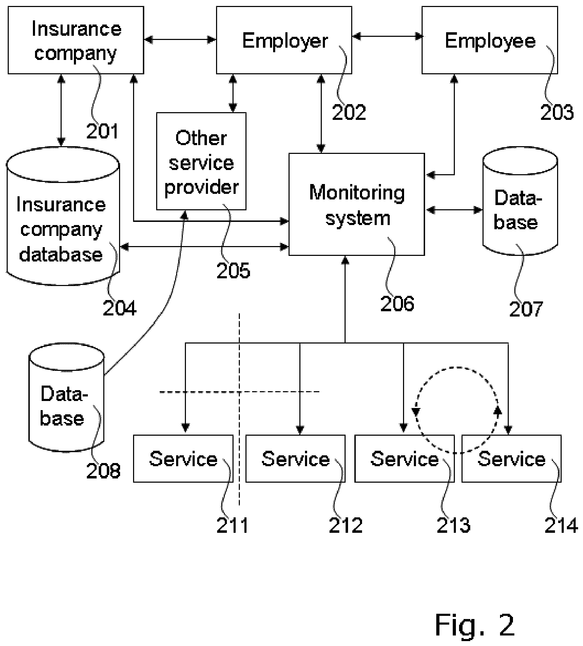

FIG 2 illustrates the monitoring system integration to the company's 121 system and the relating connections. The company 121 and the insurance company 111 providing the data base 101 to the company can be connected, as the arrows in the FIG 2 also indicates there between the company and the employee of the company 121 for instance. There is also a database illustrated for the monitoring system, so the monitoring system can store information in raw format in suitable part, meta-data, as well as observables with the status information in suitable part. Several search scripts and/or filters as well as statistical algorithms can be stored in suitable part in the memory of the monitoring system, or in the data base storage.

Below the monitoring system indicative box, there are illustrated services served by several arbitrary service providers. The way of drawing demonstrates that there are certain service providers, whose data bases can be utilized in the monitoring system in an interactive way, even so that the databases may be cross accessed, of course with legitimate rights. The FIG 2 also demonstrates service providers with their data bases accessible to the monitoring system, but separated so that the service providers do not know from each others at all, not at least via the monitoring system or via the company 121.

FIG 2 also illustrates that the company as well as the employer can access according to a respective embodiment to the monitoring system from its own perspective.

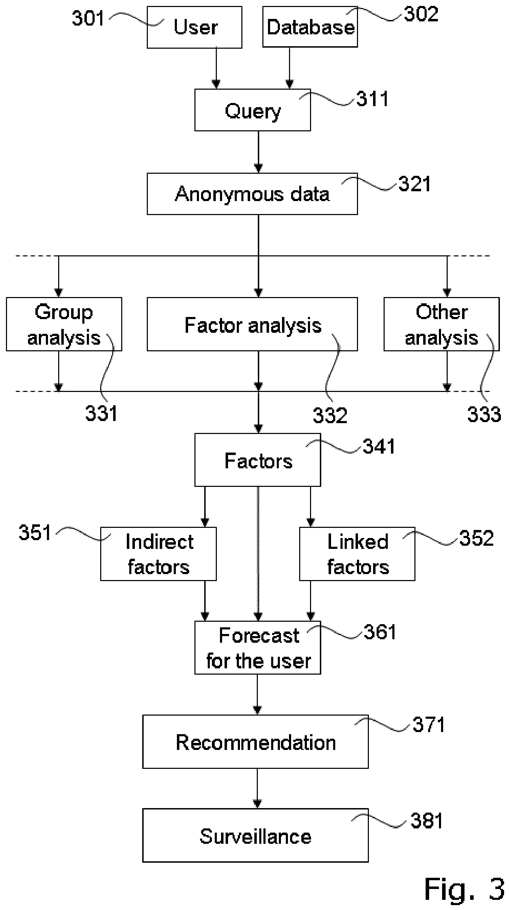

FIG 3 illustrates monitoring system according to an embodiment of the invention. At the left, a user of the system is indicated to have a query from the monitoring system. At the questioning part the system is used for creating a search or query from data base to catch observables that are under interest of the user, with the attributes or features that constitute a state or status to said observable. The features may be time dependent, indicative by a trend, or they may depend on location, hierarchy level of the observable or other variables. Thus, in each data base there might be more than a mere one entry per observable at the moment, or the other databases can be used to collect more information to the monitoring system to provide a more complete status of the observable. The observable is made anonymous at least in the monitoring system, so saving the identity unknown. The anonymity may also help getting new data bases mounted to the monitoring system. With a reference to example 4, on an observable individual and the discretion with the shown allotment of the status of the observable at a given moment, similar observables can be searched from the data bases at the moment, so as they were as cross-wise cross section of the data points available. Similar way, also trends for the observables can be searched in length wise cross section of the data points available, for each data item, or for a restricted number. The criterion in the length wise direction can be also classified to certain classes for statistical analysis to be applied later. The box anonymous data illustrates the data so searched and mined by the data mining means of the monitoring system, and made anonymous.

The data so obtained or the meta-data of the mined data can be exposed to statistical analysis for further classification, artifact removal, etc. processing and filtering for classification as indicated for the factor analysis. The factor analysis as such is not only facility available, but according to an embodiment of the invention the statistical filters and other tools as well as group analysis tools can be added to the monitoring system for improvement the analyzing diversity. Also different probabilistic tools to weight the data item of the status can be used as well as scoring algorithms in the system to score data items and/or the values according to a scoring pattern specified for the search of observables. As the factors acting in the history data for the observables are recognized, a dynamic observable can be formed with the appropriate system status, and in an embodiment as provided with a statistical shadow indicative on the statistical reliability of the features in the status. Trends are searched, by the data mining means by combining data from various sources, comparing and scoring. Direct connections as well as indirect ones can be recognized as well as the connection mechanisms revealed between features.

The user is allowed to "play", experiment, to get a prediction with the data so that "what if' in certain factors acting in the search are set differently, and so that the prediction of the trend for the dynamic observable obtained on the history basis at least partly. In an embodiment of the invention the features of the status can be valuated by points for the dynamic observable so that in an embodiment differences in the number of the points can be searched and optimized. The connection between factors can be searched, and the linking mechanisms strength can be studied.

According to an embodiment the points are indicative of monetary value, and the monitoring system can be so used in monitoring of personal trends or wellness of the personal, but not only in a company but also as a service to be sold to several other companies according their standpoints. The prediction can be used for a recommendation to improve the personal wellness for example, and the fulfillment can be monitored by following the dynamic observable time trends for the desired features.

According to an embodiment the length wise analysis of the features of the dynamic observable can be stored to the data base of the monitoring system for surveillance of the fulfillment of the trend as such, or, to be used to restrict the data for a next search. The items of the system status can be followed with the monitoring system for a particular dynamic observable via the time trends features, for instance.

According to an embodiment the monitoring system is configured for a business user. According to an embodiment the monitoring system is configured so that a legitimate employee can use the system for his/her own purposes. According to an embodiment of the invention the monitoring system is configured so that it can be legitimately used in a service provider, for instance in a personal company for improving its services.

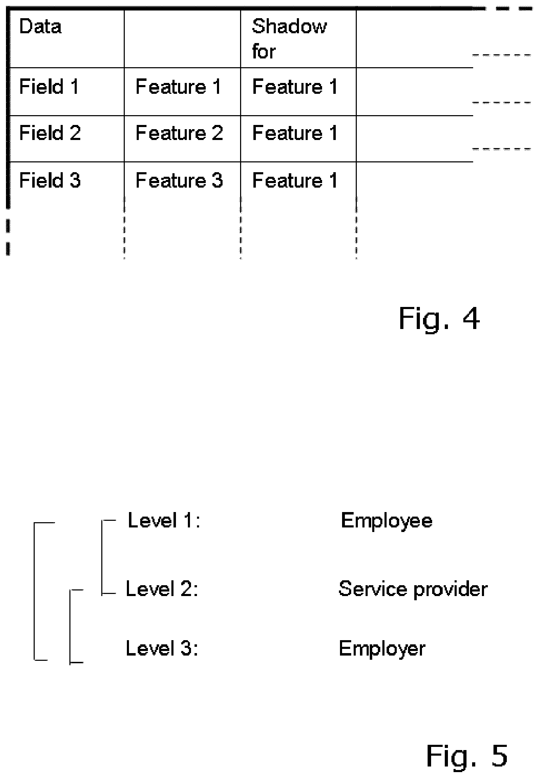

In FIG 4 different fields of the status are demonstrated as well as the nature in the example to have time dependence for the fields. Each field may have also similar shadow with corresponding time variation, or other dependence.

In FIG 5 medical services selling service provider for employers' for their employees is illustrated as they can supervise each others by using actually the same monitoring system, even as via same access point/server, each for own interests.

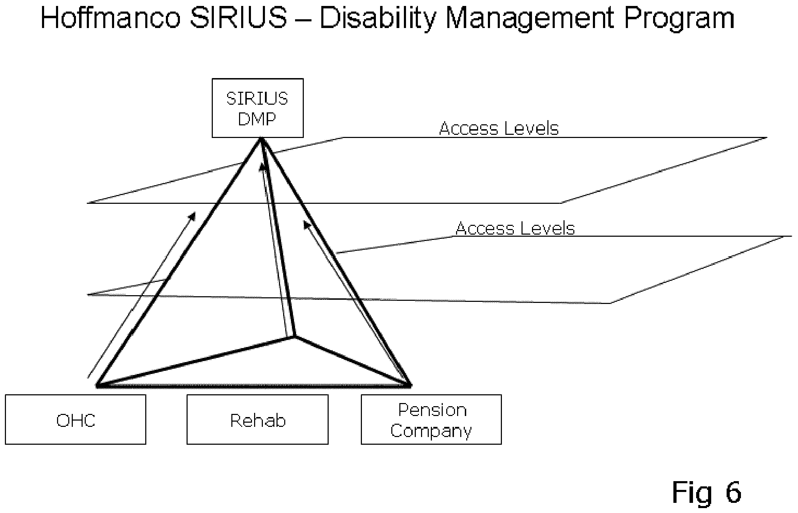

FIG 6 illustrates SIRIUS Disability Management Program (DMP) according to an aspect of an embodiment of the invention. SIRIUS collects key data from different databases; filter it to protect intimate rights and gives access to parts of it to central officers at need-to-know bases.

FIG 6 illustrates two different access levels to company officers to filtered steering data for the SIRIUS DMP in respect to OHC, Rehab Pension, and Company.

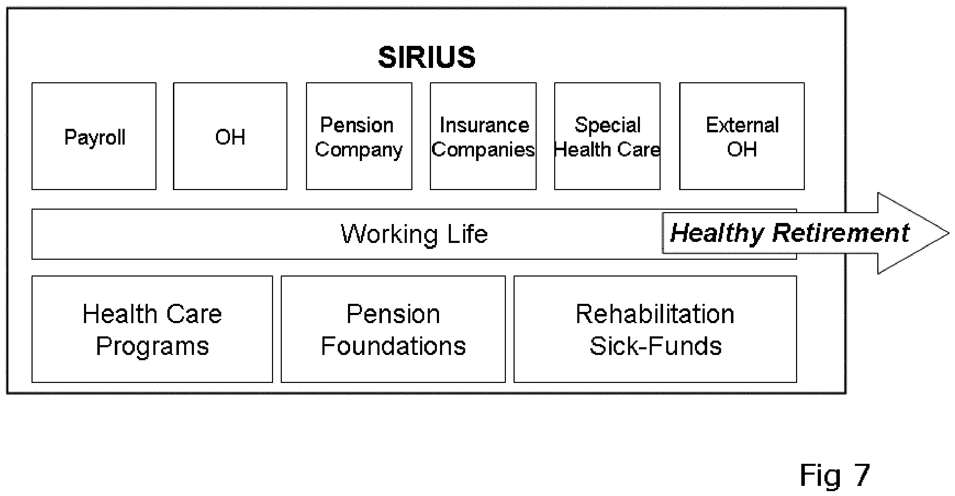

FIG 7 illustrates SIRIUS at micro level. According to an aspect of the embodiment, key steering data, needed for efficient management of both present and imminent disability in an organization, is widely spread among a large number of non-communicating data bases. However the SIRIUS program connects key information from separate data bases in a protected way to enhance decision making. For healthy retirement after the working life of an individual, the SIRIUS uses in the example data from Payroll, OH, Pension Company, Insurance Companies, Special Health Care, External OH, Health Care Programs, Pension Foundations, and/or Rehabilitation Sick-funds.

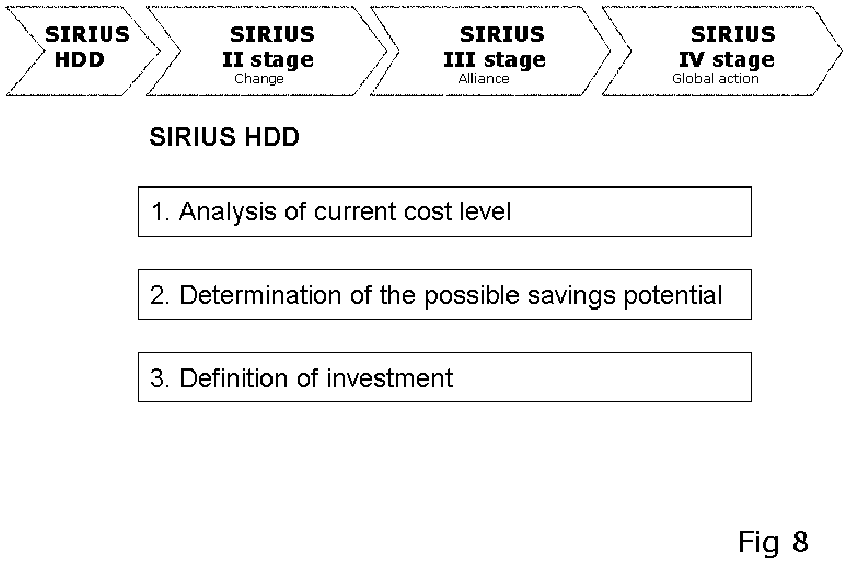

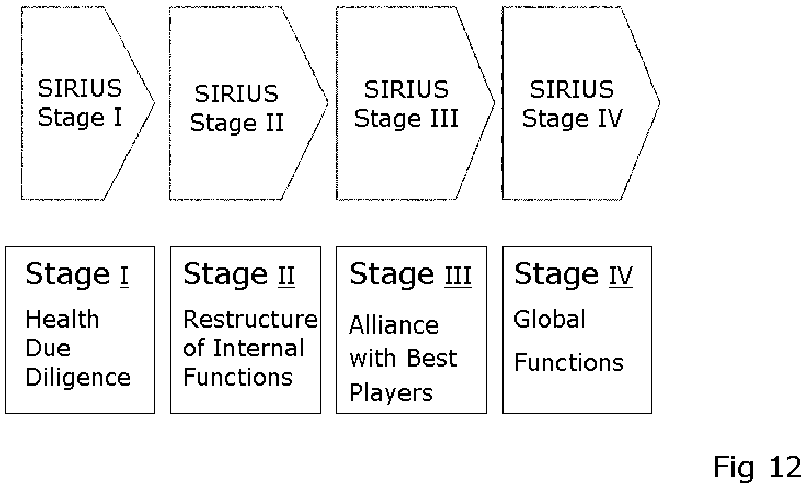

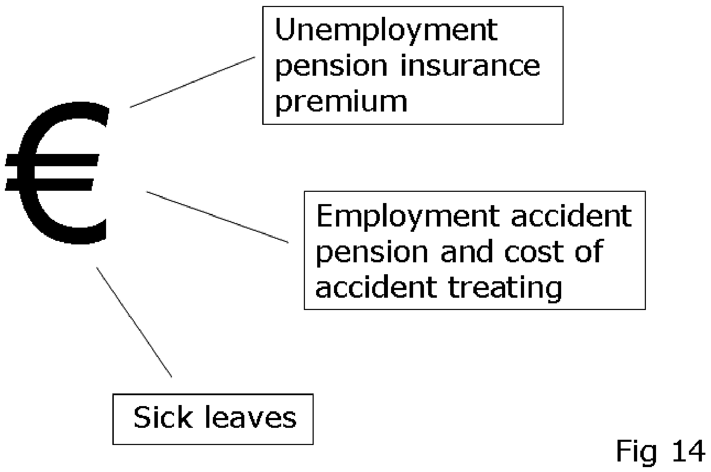

FIG 8 illustrates aspects of SIRIUS according to an embodiment, with reference to Figs 12 and 14 for the heath due diligence HDD. The FIG 8 illustrates SIRIUS stages I, II, III and IV, HDD, Change, Alliance and Global action, respectively. According to an aspect of embodiment, the SIRIUS HDD relates according to FIG 8 to analysis of current cost level and the reasons for it is utilized, Determination of the possible savings potential with the SIRIUS in national / international level and to definition of the necessary investments.

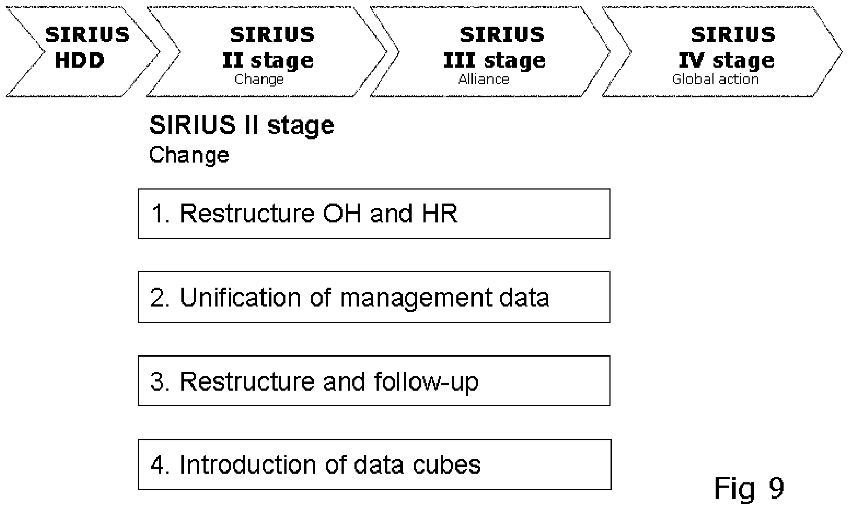

FIG 9 illustrates II stage in SIRIUS according to an aspect of an embodiment. The II stage Change comprises: restructure of the OH and HR co-operation, Unification of essential management data through SISIRUS, Restructure and follow up of OH functions and Introduction of SIRIUS Data Cubes as tools for Disability Management.

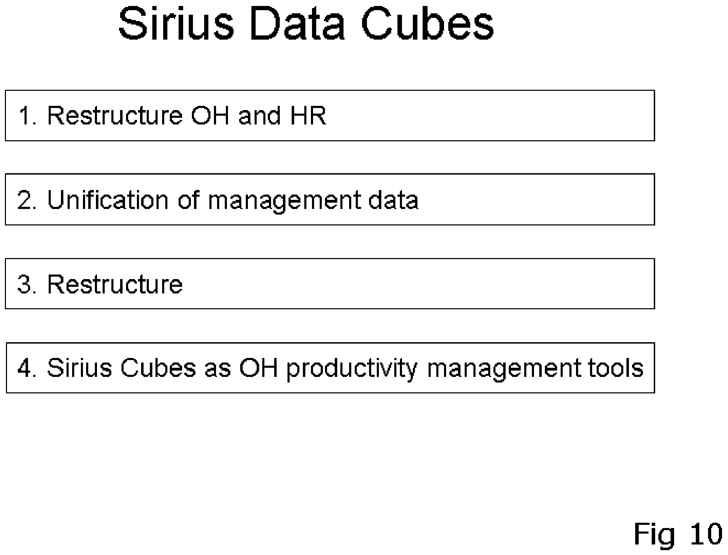

FIG 10 illustrates SIRIUS Data Cubes in relation to an aspect of an embodiment. Accordingly OH and HR cooperation are restructured. Essential management data can be unified through SIRIUS. OH functions can be restructured, as SIRIUS Cubes function as OH productivity management tools to guarantee optimal treatment of serious illness. Accordingly, Costs can be saved as disability avoided as for example, because of a timely endoprsthesis treatment, Proper treatment of RR patients - normotonic 72 % (Finnish average 20-25%), Diabetes type II normoglycemic 87 % (average in Finland DEHKO Apr. 30%), Depression treatment will start in 2-6 days after diagnose (normal in Finland after 32 days) with average sick leave of 6 weeks (average in Finland 6 months or more), special subsidy in pharmaceuticals (up to 80% of costs).

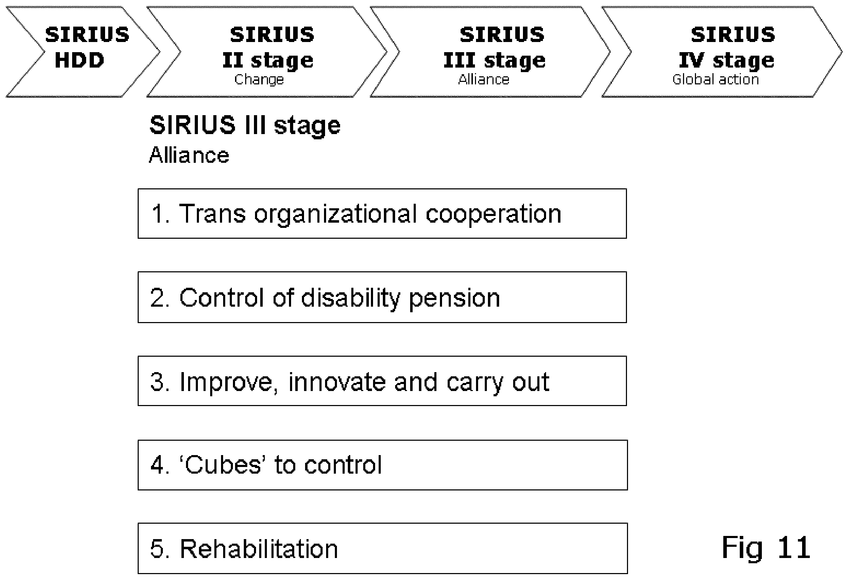

FIG 11 illustrates SIRIUS III stage. According to an aspect for an embodiment, Trans-organizational cooperation within essential support functions can be provided, as well as control of disability pension procedure and the costs. SIRIUS can perform also as a base for improvements, innovations and carrying out new working methods for the real results. SIRIUS can allow moving out from soft nursing to efficient surgery and treatment with constant focus on end results, as cost of timely treatment is a percentage of the cost of disability. The cubes can control work and enhance timely rehabilitation.

FIG 12 illustrates an example on an embodiment of SIRIUS utilization on European level of competitiveness with the SIRIUS -project according to the stages I, II, III and IV comprising health due diligence, restructure of internal functions, alliance with best players and global functions, respectively.

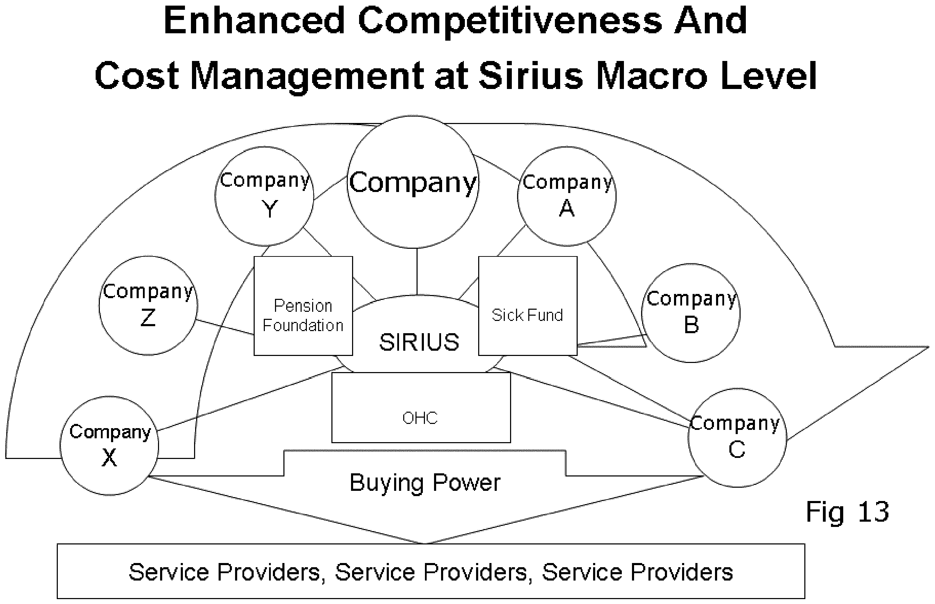

FIG 13 illustrates SIRIUS at macro level. According to an aspect of embodiment SIRIUS relates to Corporation, OHC, Pension Foundation and Sick-Funds as illustrated in FIG 13, but also to enhanced competitiveness and cost management. SIRIUS can also relate to several companies X, Y, Z as well as companies A, B, and C to yield buying power for service providers of several types as illustrated by the repetition of service providers.

FIG 14 illustrates aspects of Health Due Diligence ^{®} (HDD) for determination of a working ability administration in a company, especially for leak-out costs in a large company's as an employee. The success of administration and control of: 1 TyEl-insurance fee depend on the classification as 19.2 -31 % from the salary costs. 2. Accident originating retirements and accident occurrence treatment costs, 3 Absence due sickness, that provide the amount of the balance that is leaking out of the company.

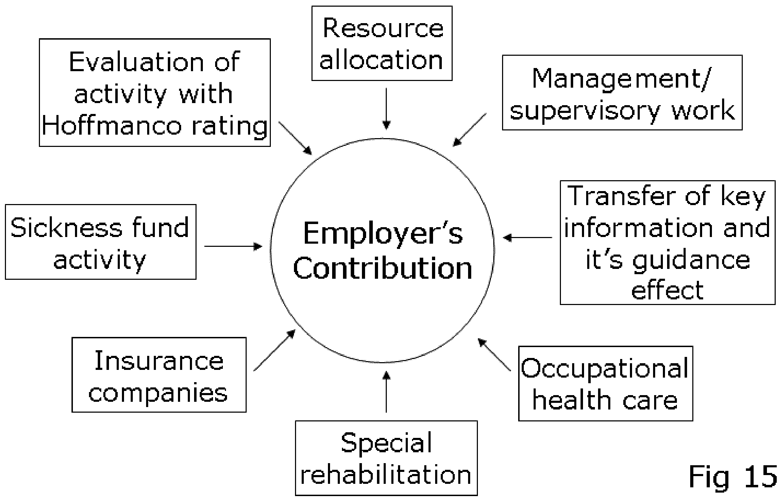

FIG 15 illustrates aspects on point of views for a HDD-mapping to be considered and the potential savings provided and the actions for them. Large entity employer costs, that the employer can itself influence are TyEl-insurance fee - the unemployment-part, accident originating retirements and accident occurrence treatment costs,

The employer costs are affected by Resourcing, Leadership/supervisor work, key-information transference and its guidance, working health care, special conditioning, insurance companies, sick cash activities and evaluation of activities according to the Hoffmanco-point-scale.

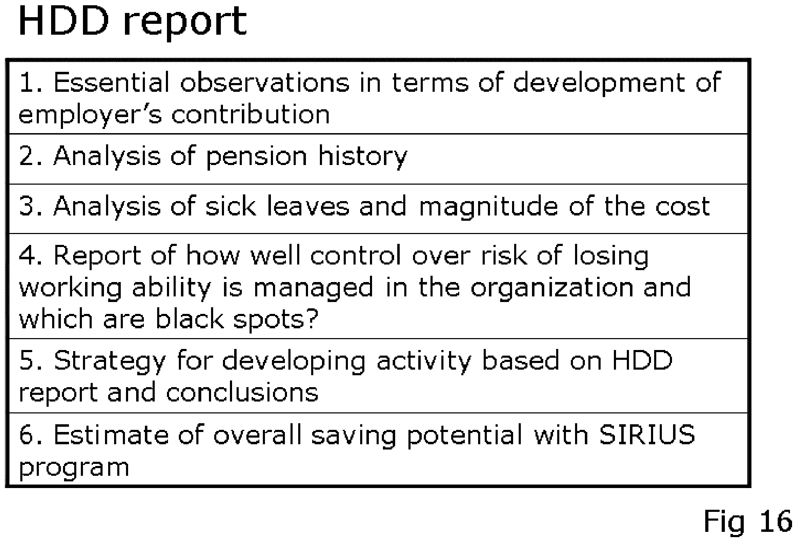

FIG 16 illustrates reporting aspects according to an embodiment for areas of health due diligence, the employer costs formation and reasons. 1 Essential observations on employer costs formation, 2 analysis of the retirement history, 3 analysis on absence due sickness and the consequent costs the magnitude thereof, 4 Explanation on that how well the administration of risk of working ability loss has been lead in the organization and what are the problematic aspects therein, 5 Working plan for improvement of the activities in accordance of the HDD-report and the conclusions. 6 An estimate on the total savings potential according to the SIRIUS-program code. HDD-report provides the substantial information for initiation of a development process in the company.

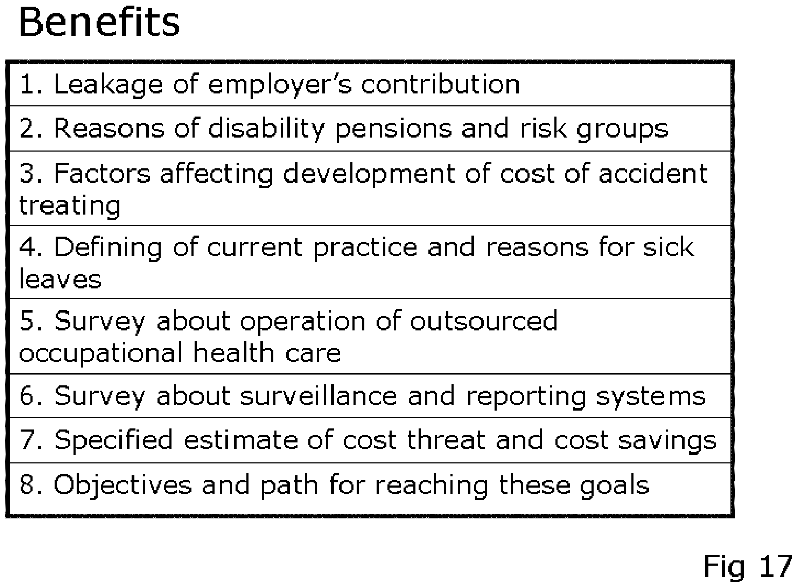

FIG 17. illustrates potential benefit aspects on Health Due Diligence. With the HDD a company can localize the employer costs that leak out 1 and identify the reasons thereon, as without this particular piece of information concrete actions on the leak-outs are difficult to be intervened. Cubes on working disability clarify work disability reasons and risk groups 2; on the basis of this information risk group mapping and risk group being taken care can be made and remarkably influence on the administration of working disablement risks in the future. Cubes on working disability provide a clarification on accident treatment costs formation and the factors there behind 3, enabling to see how to systematically reduce insurance fees. Absence due sickness, determination in respect of the current state and the reasons for them 4 constitutes a facility to develop working ability management supporting routine for following the absence due sickness that supports successes in working health care activities, and supervisor activities. Additionally HDD constitutes a way to direct and manage in a centralized manner the working health care activities of an external unit.

Mapping of activities of an outsourced working health care 5 create a vision on the real potential of the company to support the employee's working ability and strength, manage working disability retirement risks and safeguard the cash. Mapping of following and reporting systems 6 creates a vision on the potential of the current systems to support good working ability tracing and management, localization of deficiencies creates potential for development of a watertight working disability control system. On the basis of the HDD an accommodated calculation 7 can be made on the employer costs and the working disability risk induced cost threads and the cost savings potentially gained. On the basis of localized and indentified problems the goals and the path to meet the goals 8 can be defined for a company for the working ability guiding system.

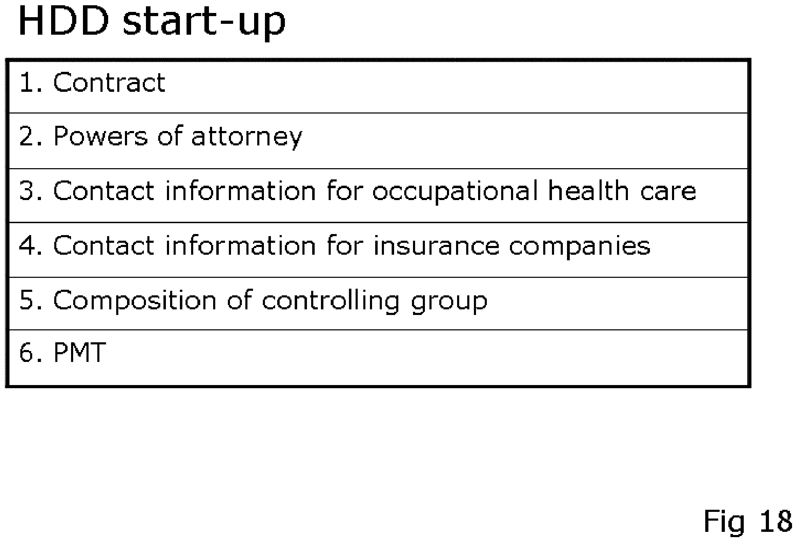

FIG 18 illustrates aspects of embodiments on HDD initiation. At the HDD initiation, contract is made, power documents, working health care communications information, insurance companies' communications information, composition of the Control group and the PMT.

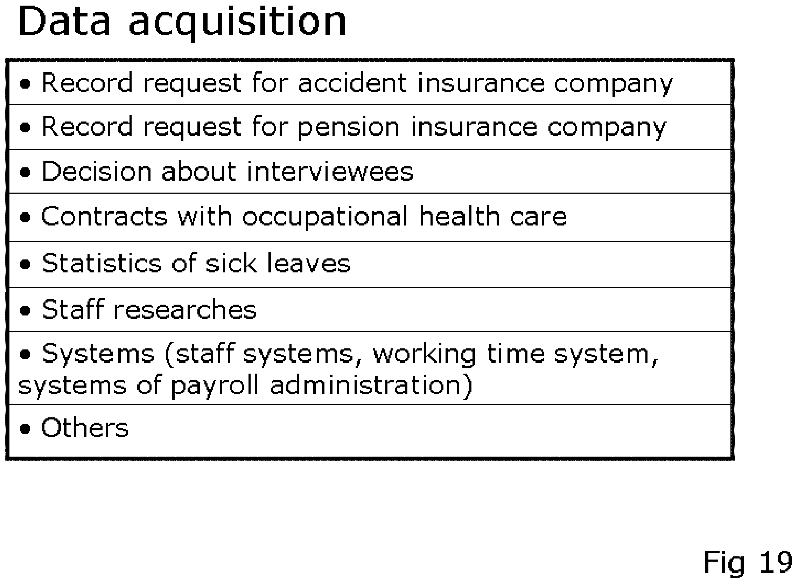

FIG 19 illustrates aspects of embodiments for information acquisition relating aspects on accident company's request on recordings, retirement insurance company's request on recordings, deciding the interviews, working health care contracts, absence due sickness statistics, personal studies, systems (personal systems, - work time systems, salary administration systems) and other aspects.

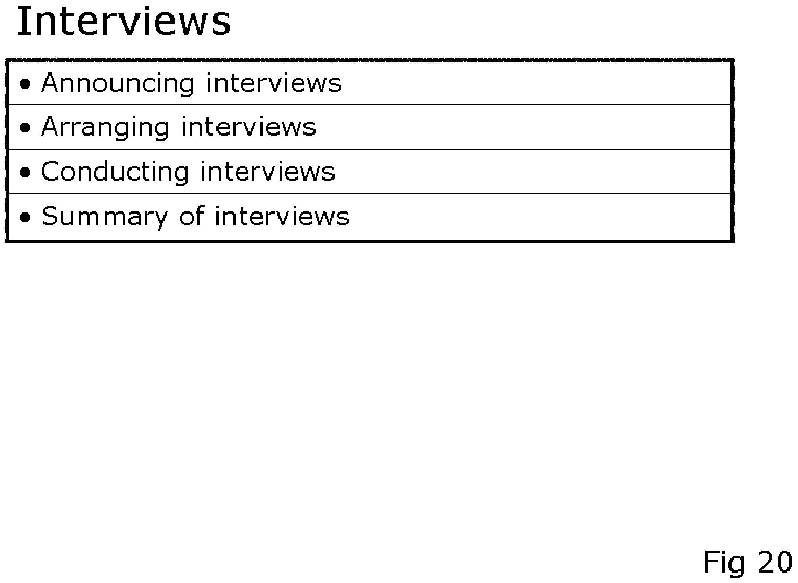

FIG 20 illustrates the aspects of embodiments for interviews, the announcing of them, contracting, making and concluding them

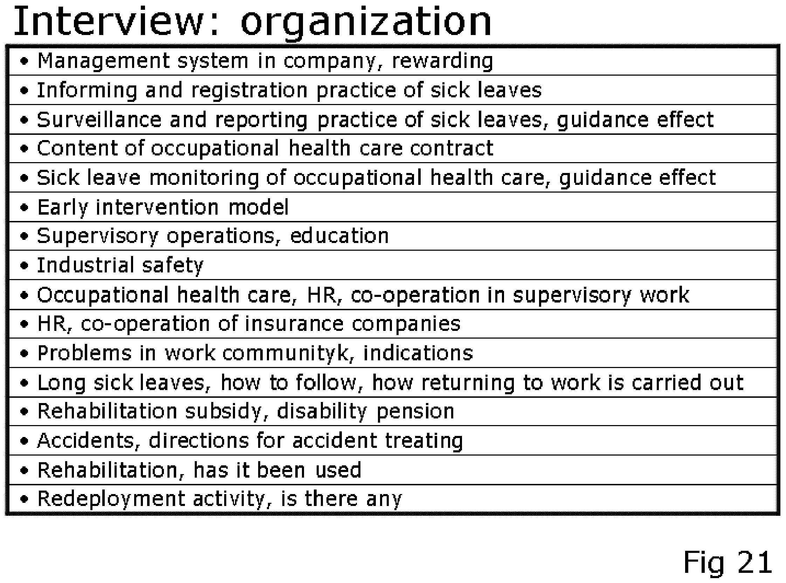

FIG 21 illustrates the aspects of embodiments for organization interviews. Following aspects are shown: Management system in the company, rewarding, Absence due sickness the methods of informing and reporting, Tracking of absence due sickness, reporting , the controlling effect, Working health care contract content, Absence due sickness tracking the controlling effect of the Working health care, Early intervention model, Supervisor activities and training, Health and safety in work, Working health care, HR, supervisor co-operation, HR, insurance company co-operation, Problems and symptoms in the community, Long absences due sickness, how to trace, how to implement returning to the work. Conditioning support, working inability-, tk- pensions, Accidents, the instructions on the activities therewith, Conditioning, has it used?, Rehabilitation activities do it exist.

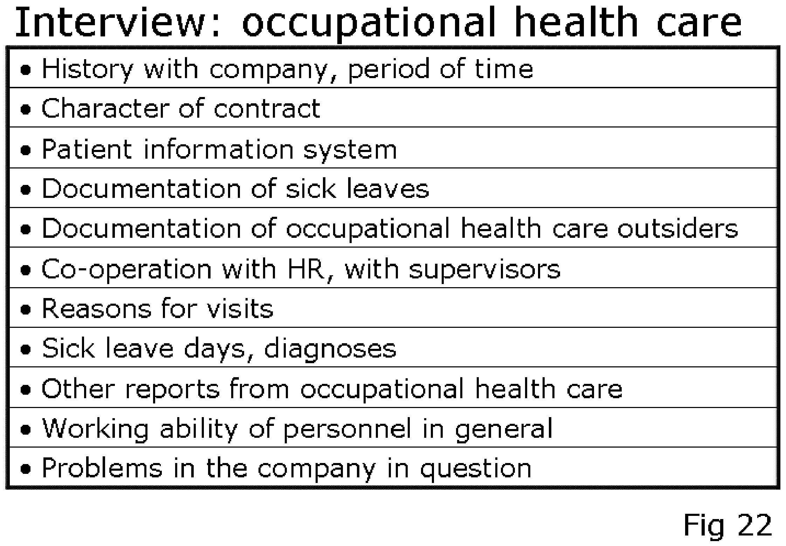

FIG 22 illustrates the aspects of embodiments for organization interviews. Following aspects are shown: Background within the company, the period, Contract characteristics, Patient information system, Booking of the absence due sicknesses, Booking of the externals to working health care, Co-operation with HR, with supervisors, The reasons for the visits, Sick leave days, the diagnosis, Other working health care reports, The overwhelming working ability in the company, Problems in the company.

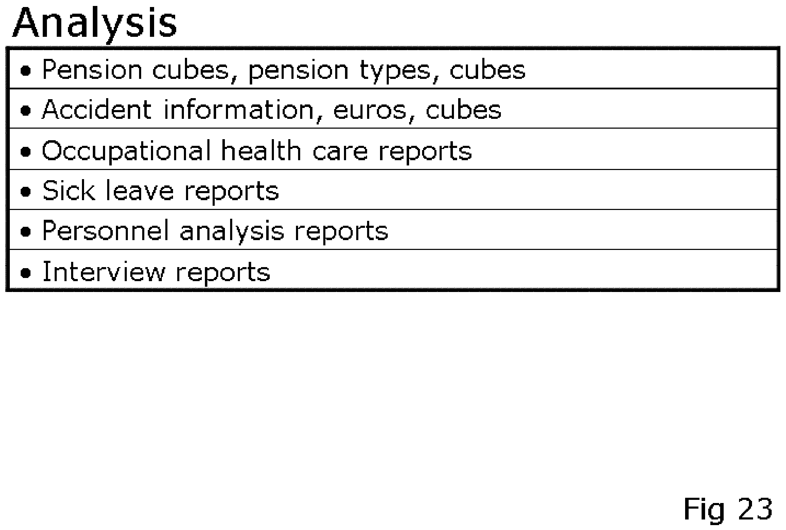

FIG 23 illustrates analytical aspects on embodiments. Retirement cubes, -species, cubes; Accident information, Euros, cubes; reports of the working health care; Reports on absence due sickness; Outcomes of personal inquiries, Outcomes of the interviews.

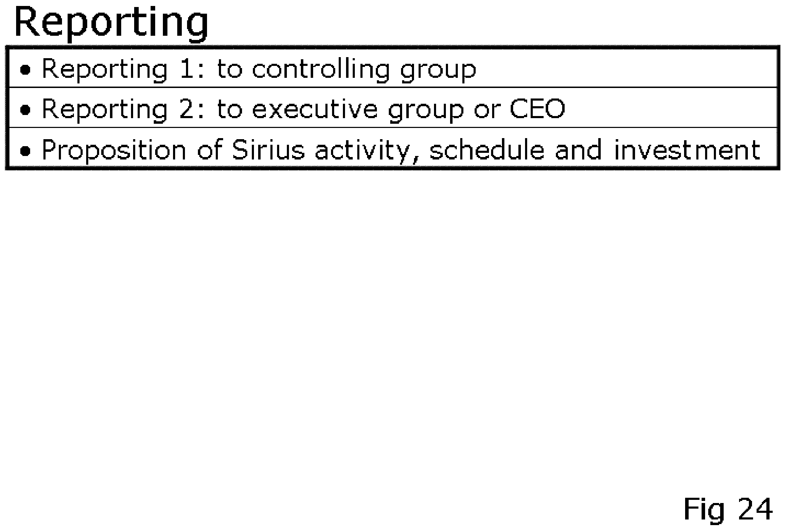

FIG 24 illustrates reporting aspect on embodiments. Reporting to control group, leading group or to the CEO, SIRIUS-act suggestion, timetable and the investment.

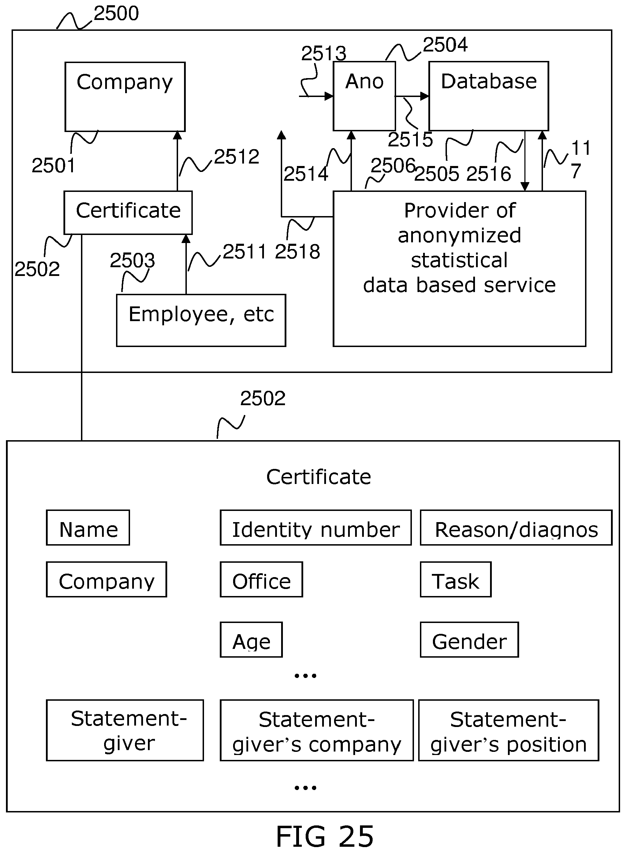

FIG 25 illustrates the utilization environment of the anonymizing interface and the use. FIG 25 shows a arrangement 2500 for example in a working community of an enterprise 2501 on whose employee 2503 there has been made 2511 a certificate 2502. The certificate 2502 can comprise in suitable part a diagnosis and/or information on the inability of the employee-permanently or according to a predefined temporal period, but it can comprise also other details and/or reasons on the theme of the certificate. The information can be in readable words, but also in coded words. When the employee 2503 or an entity devoted to deal his/her matter delivers the 2512 the certificate in a form to the employer 2501, it archives it into its own information system, that comprises information on the employee, his/her health status, diagnosis and the identity. According to the known techniques, the information keeps in the file classified and nobody else can use the information. In such a case an outsourced service provider has to accept the situation and provide services accordingly, although the aim of the service were on the wellness of the working community, and consequently also on that of the employee. Such a service provider is referred as a service provider 2506.

In order to solve such a problem, the enterprise 2501 and the service provider 2506 agree on maintenance 2518 of data base services for the needs of the enterprise, according to an embodiment of the invention, wherein the data base services comprises the maintenance of the data base 2505 and/or the updating, in regard to the employees 2503 of the enterprise 2501, according to the cumulating information on working ability, health, sicknesses, or other information details in the health status of the employee, for instance, according to the scope of the agreement between the service provider and the employer.

According to an embodiment of the invention the anonymizing interface 2504 can be implemented when the service provider 2506 wants to analyze the heath data on a statistical scope. The anonymizing interface 2504 has a task to anonymize the information 2502 on the employee 2503 to anonymous statistical information and to convert the information 2513 on an individual 2503 into such a form where the heath information has been saved as such but the identity of the employee or another corresponding individual is not any more identifiable for recording 2515 in the maintenance of the service provider's 2506 and/or enterprise's 2501 data bases 2505, in the anonymous form of statistical information as defined in the information protection criteria 2514. Upon the recording, it is possible to evaluate that in the appropriate classes there are sufficiently many anonymous recordings on the individuals to guarantee not to compromise the anonymousness of the persons in the light of the information protection criteria. In such a case for instance, according to such an exemplary criteria, if five or more individuals were in the same class, the information has a statistical character rather than heath information. According to an embodiment of the invention the anonymizing interface 2504 is configured to mark into a certain record a utilizability attribute so that the data base can pass such records that have the marking indicative for non-utilizability for a certain field or fields in the allotment.

Thus, within the service provider's 2506 provision 2516, 2518 the employer can search information from an anonymous data base 2505, or if so agreed on between the employer enterprise and the service provider, also information directly from the data base 2505. Additionally the service provider 2506 can use 2517 data base in suitable part as agreed on, for its own purposes, and/or maintain and/or update the data base.

The operation of the anonymizing interface 2504 can be illustrated by the enlarged view on the certificate 2502.

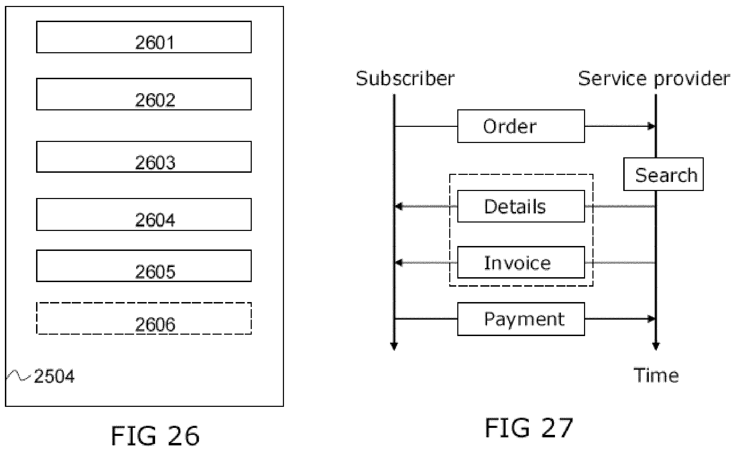

In FIG 26, the drawn boxes inside the certificate 2502 illustrate fields of the certificate 2502. The number, content or any other detail of the fields are not limited to the mere shown example. The certificate 2502 can be for example a routine examination certificate, sick leave certificate, disablement certificate, retirement action, certificate on an accident, or another document 2502 reporting on the physical and/or mental state of the employee, not limiting as such into any particular or a combination thereof only. The document 2502 can be a paper document, so that the entry into the data base 2505 can be implemented by feeding 2513 the information via a reader or by feeding the information manually into the system, comprising the anonymizing interface 2504 arranged to be operable at an interface of the system. According to an embodiment of the invention the anonymizing interface 2504 can comprise a scanner and/or a text recognition means arranged to recognize the fields and/or their content.

According to an embodiment, the document 2502 can be a record in a file or data base or another corresponding part thereof that is machine readable 2513 via the anonymizing interface 2504 into a data base 2505, so that the anonymizing interface removes the original identity from the record or another corresponding part that is recordable into the data base.

According to an embodiment of the invention the anonymizing interface 2504, 2604 comprises means for recognizing 2601 a field that comprises a name, and/or means for means for recognizing 2602 social security number (Sotu). According to an embodiment of the invention the anonymizing interface 2604 comprises also means 2603 for reading 2513 other fields that are illustrated by the boxes in examples and/or figures. In this context a reference is made also to FIG 25 showing the information in the fields also illustrated by the boxes, in accordance of the examples given later, in suitable part. The means for recognition of the anonymizing interface can be in suitable part diversely placed or optionally mutually integrated together. The fields that anonymizing interface 2504 has been configured to machine read for recording 2515 into the data base 2505 are read and recorded, but the field that contains the individual's name and/or social security number or another corresponding entry containing field can be recognized to contain such information, but is not recorded into the data base 2505. According to an embodiment of the invention the anonymizing interface 2504 can be arranged to give a temporal identity to a record according to the person it concerns, if it were under suspicion a serious misuse, danger of death, or another serious fact supporting the later identification of the person, and it is not clear what to do when reading the information in. In such a case the anonymizing interface 2504 can be provided with a special means 2604 which is configured to provide the record with a temporal identity into the data base 2505, but arranged to communicate the temporal identity also into the own system of the enterprise 2501, from which the information in accordance of the original identity came from, on the said fact and on the new identity. In such a case the temporal identity is known by the employer, as all its already sent information. However, the temporal identity hides the true identity from the service provider, but in a problematic occurrence the enterprise and the service provider can together deal the same data in suitable part via the original identity and/or temporal identity, but without a danger to leak the identity of the individual to an entity not allowed to know it.

According to an embodiment of the invention the anonymizing interface 2504 is arranged to give a temporal identity in such cases in which there are too few persons in a same class to full fill the information protection criteria for the information handling as statistical information. In such a case the anonymizing interface 2504 can be configured to remove the temporal identities from such records that comprise sufficiently numbered amount of anonymous pieces of information in the data base, thus regarding the information having a statistical nature. According to an embodiment of the invention the interface keeps record on the content of the data base and/or classifies the given temporal identities.

According to the configuration 2514 of the service provider 2506, the anonymizing interface 2504 comprises a clock 2605, according to which the temporal identities can be nullified after a certain period of time, so to safeguard the identities. So the employees' identities can be kept secret, but still have the information for use in statistical analysis. The information can be used for good of the enterprise 2501 for the wellness of the employees, but as well also in the optimization of its own retirement outcomes. According to an embodiment of the invention the anonymizing interface comprises an interface-driver for combining a data acquisition device to the anonymizing interface. According to an embodiment of the invention the anonymizing interface comprises a hard-ware part, that has a data acquisition device and/or a reader for reading information as input for the anonymizing interface, the device driver and/or the device has been illustrated with the reference number 2606. According to an embodiment of the invention the hard-ware part has been configured to operate as a platform to the software part of the anonymizing interface, which is configured to configure the hard ware part to provide the functionality of the anonymizing interface. According to an embodiment of the invention a certain software means for providing and/or assembling the functionality of the anonymizing interface 2504 are arranged into a machine readable form on a machine readable saving media, from which the execution the software part of the anonymizing interface is set up to be utilizable in the operation defined the hardware part. According to an embodiment of the invention the hard ware part comprises a reader and/or functional reader that are connected to an outer information network at its interface.

FIG 27 illustrates an advantageous business-related method in accordance of the embodiment of the invention, as a non-limiting example for an embodiment in which as a subscriber acting enterprise subscribes statistical information on the health related details of its own employees. In FIG 27 the subscriber sends via information net work /gives otherwise the subscription to the service provider on the information and/or combinations thereof. The service provider who received the subscription makes the search from the data base that is in its disposal. The service provider sends as a response - either in same connection with the invoice or separately as the subscription related information according to the subscription to the subscriber. Because the delivery of the information and invoice can be made essentially simultaneously or separately, these embodiments are illustrated so that the simultaneous delivery comprising response combination embodiment is illustrated with a dashed line. Although subscribing, search and delivery and payment are illustratively shown in a certain order, it is clear from the embodiments of the invention to a skilled man in the art that subscription, search, delivery and the payment can be arranged to occur in almost any order, with the provision that the content of the subscription exists so defining the parameters for the search. In such a case, the subscription can be a one time subscription, but in suitable part such a subscription that is sliced, intended to be performed within certain parts, and/or intended to be performed on open until further notice- principle. The payment can be addressed also to either on the time interval context or on to the partial delivery context, ether in advance, within the delivery, or after the delivery, in suitable part. According to an embodiment of the invention the subscription defined information can be scaled for the delivery into a larger frame of a higher level entity, when the frame of the higher level entity as whole full fills the information protection law criteria so that the statistical information under delivery do not comprise indication on/identities from other companies and/or on the individual employees or other individuals. Although the vertical line is marked as time, and the horizontal lines are horizontal within the readout accuracy, there is a time, there between each sending and receiving moments for the subscriptions and deliveries, corresponding the travelling time of the message, which time is not illustrated in the drawing for sake of simplicity.

It is apparent to a person skilled in the art that as technology advanced, the basic idea of the invention can be implemented in various ways. The invention and its embodiments are therefore not restricted to the above and/or below shown examples, but they may vary within the scope of the claims.

### Example 1

A company operates as a supervisor via an executive user, having in use a monitoring system according to an embodiment of the invention for management of the decision making in the company ,or enterprise. The company has bought an insurance service from a local insurance company for the personal of the company, as well as certain other services from other local service providers, the number or quality of which is not limited only according to the example. The company has agreed with the insurance company that insurance company serves an access to the company also into the data base for the company for information to be browsed so that the company can observe the data that is available on the employees of the company for instance for the accidents and/or sicknesses.

According to the example the company would like to know a prediction on the number of persons that retire in next 5 years being predicted according to an embodiment of the invention. Thus, during a session of the company representative, the monitoring system accesses to the data base of the insurance company and the data mining means of the monitoring system is allowed to access to the insurance company's data base with a provision of an appropriate security limitations, suitable for searching particularly files of the company relevant records on the employees that are ready to retire in the next 5 years. As there might be age, and health aspects expected as well as number of years in duty, the data mining means in the system can have a simple Baylesian filter activated for the search, but is not limited only use of such filter, the search is configured to pick up persons that match to the retirement criterions of the Baylesian filter or another criterion for the management to produce control data.

According to the utilization of the data base facilities, also other features for the persons are reached. The desired features are pre-defined according to a scheme arranged beforehand for a routine search like this. The user interface comprises also means to refine and/or create searches as well as means to mount/unmount databases into the monitoring system.

A factor analysis means search common information items available for the found persons that match to the retirement criterion, and factorizes the items for finding the underneath reasons for retiring. As the company in the example comprises tens of thousands of employees, there would be representative number of the retiring employees. According to an embodiment of the invention the intermediate data that is mined from the insurance company's data base is made impersonal by an anonymizing interface and thus the personality of the employees is maintained anonymous, and the privacy is saved.

As the insurance company has records on the health information for each person that has been dealing with the insurance company, the information on the persons can be considered as data items that form data for statistical analysis on an anonymous basis. The data mining means thus can arrange the found persons according to the profession and/or particular tasks for example or into groups so that for example number of visits for medical experts as well as their previous treatments is shown in detail, or other criterion can be used to as such for the information. In a variant of an embodiment also the medication as well as the dosage has been shown as a trend according to the history information available from the insurance company's data base. Correlations are searched there between trends.

In the example, not all the data are available from the insurance company, but also records of a medical system provider used by the company for its employers is made accessible, so the data mining means can search records according to the found persons from the insurance company, who are treated as such as anonymous so that the true identity, although were available is not revealed to company or its representative, nor to the service providers in the example.

According to the information available from the number of sources, in the example insurance company and medical center - not limited only thereto, the interface converts the acquired data to ensembles that retire in 1, 2, 3, 4 and 5 years. The ensembles are characterized in the report by common features that were found by the data mining means. So, the company can see that certain groups have special features, for instance, that the blood pressure average in a group correlates with an early retirement when there is also sick leave between certain lower and upper limit of days in a year. The correlation mentioned is only illustrative, not restrictive to the mentioned only. The observables and the corresponding status can be identified for forming dynamic observables for the corresponding years.

According to an embodiment the data mining means are re-organized in the control of an analytical unit having an access point and a user interface for the executive user, so that the mining process can be made again taking into account the new observation on the sick leave days and blood pressure. Additionally there might be additional data bases that could be used in the mining process, and the user selects also another health organization used in the company's concern in another business, and adds the data base for a revised search.

According to an embodiment of the invention the ensembles are set as objects that have the system status according to the features associated on the basis of the data mining and further processed with anonymizing interface into the form in which the system status for the ensembles as observables are shown in a server for example.

According to an embodiment the system status is shown at a server of the company. According to an embodiment of the invention the system comprises also said server, or at least an interface and/or the anonymizing interface to the system so that the mining events can be accessed and/or revised in respect of the data base selection and/or the criteria according to a pre-defined scheme or according to a modified scheme derived from a pre-defined scheme.

In the company the responsible executives have a terminal device with an access to the data bases under the monitoring systems surveillance and command. As the user notices that certain personal history in average for a particular ensemble leads to certain out come for the members of the ensemble, the user would like to experiment with the system for optimizing the retirement of the ensemble for the management on the basis of the data just produced, and thus would like to see how certain choices control the reteriment in the company. Certain actions in the expected event history in the future are weighted and/or scored in combination with probabilities of what would happen so that the outcome change can be observed in respect to the unchanged or other reference scenario. In addition, there is also available an extra data base of already fulfilled retirements with full history. The estimates and probabilities for the expectation estimation can be derived at least partly from such a data base. Personal management system can thus use the monitoring system for decision making.

### Example 2.

It is as the example 1 but seen from the point of view of an employee. An employee of the example had a medical analysis made before and the diagnosis results were recorded into a data base of the system, as many as that of his/her colleagues, during several years so that the items are forming a history on the dosage of medication, accidents, weight, blood pressure, etc. for said employee. The employee knows his own data, but would like to know is the present condition sufficient for him to survive to the retirement age. Thus, the employee accesses to the monitoring system via the employee's access point and corresponding user interface. The user defines a search for the data mining means of the monitoring system by modifying a pre-defined search example for better meet his profession "clerk" for instance. The data mining means access to the data bases that are mounted for the search or mounts additional data bases so that the search can be made. The data mining means checks is there a previous search similarly made, and is there consequently suitable meta data already available, and/or should additional sources searched and/or used.

The data mining means finds the data from which the interface finds the trend of the blood pressure as well as sick leave correlations in the ensemble consisting of executives and other office workers. The user revises the data so that office workers are counted to the ensemble so better match his profession. The data mining means make a search and the details of office workers in average are shown to the user. Five most common to at least ten office workers items are selected for the display and the user can see the data items of those that have been retired.

In addition, to make sure that his vision were correct according to his own information on his health, in respect to the ensemble of already retired personal, the user also revises the search so that there are only office workers included that have slight blood pressure as the user, but died before retirement. The data mining means makes the revised search, and show again five items most common to at least ten office workers that has died before their retirement.

As the two searches as compared reveals that the exorcize has been in that company a key factor for reaching the retirement age for office workers, when too hard and too little exercise practicing office workers have not reached the retirement age, the user decides to have some more exercise in a medium level.

### Example 3.

As in the example 1 and/or 2, the scenario is the same, but the health service provider is using the monitoring system according to an embodiment of the invention. The searches as made can be the same or similar kind in suitable part, but the access point and the user interface may be arranged differently as well as the predefined search patterns, better to match the wellness aims of the service provider.

A similar exemplary search as made in example 2 and it indicated that retirement of the office workers of the client concern was increased in those companies of the concern in which medication for the blood pressure was started earlier than any real problems were encountered in visible. The wellness service provider can so take the notice into account when making recommendations to the client concern.

### Example 4.

Example 4 demonstrates via shown data element, relating to accidents for a relevant data base, actions that can relate to individual observables, but also that the data entries in the records can be made anonymous. The actions and/or accidents apparent from the example 4 can be described according to a standard way, for example codes of European statistics on accidents at work (ESAW), or another suitable code. The lengths of the records can vary, or may be left empty in suitable part. The example 4 gives also one example on allotment of the system to allot an observable, with its status of presence in the system, to be used in for a data mining process. According to an embodiment, the anonymizing interface and/or the data mining means of the monitoring system can be arranged to collect the data entries form the data bases.

According to an embodiment of the invention the anonymizing interface is arranged to read confidential information from records having an allotment as demonstrated, from the data storage of the information, so that the user can observe records in it and can recognize from said records identity information so that when said records used as input to said anonymizing interface, it is arranged to output said records or certain parts of such without the original identity in the allotment of the output information. The related system can use the embodied anonymizer as indicated in the related embodiments of the invention. The system according to an embodiment of the invention can be used for communicating the information anonymously as statistical information for a further use.

| **Column** | **Length** | **Type** | **Notification** |
|---|---|---|---|
| Unit | 35 | text | The name of the unit |
| Postal code | 6 | text | Postal code |
| Location | 30 | text | Post address |
| Insurance id | 13 | text | Insurance identification code |
| Type | 3 | text | W (working acci.), F (free time acci.) |
| Injured | 35 | text | Last name firs name(s) |
| Social security number | 11 | text | For example 010160-0XX |
| Accidental time | 10 | date | For example, YYYY.MM.DD |
| Compensated days | 3 | numeric | Whole days |
| Temporary compensation | 9 | numeric | Compensation in cents, for instance |
| Permanent compensation | 15 | text | Compensation in cents, for instance |
| Class of previously diagnosed disease | 4 | text | Main class of the disease, for example, S092, M545, M75 |
| Injury area (ES) | 4 | text | 55 = wrist, 12 = face, 14=ears, etc. (ESAW) |
| Damage type (ES) | 3 | text | 020 = broken bone, 010= wounds, etc (ESAW) |
| Working spot (ES) | 1 | text | 1= under administration of own employer, 2=under somebody else's (ESAW) |
| Task (ES) | 2 | text | 11= production, 21 = land construction (ESAW) |
| Performance (ES) | 2 | text | 10= machine operating, 30 = vehicle control (ESAW) |
| Displacement(ES) | 2 | text | 10= electrical disturbance (ESAW) |
| Way of Injury (ES) | 4 | text | 12=electric shock, 14=cold (ESAW) |
| Cause of damage(ES) | 4 | text | 1221=ladders movable, 2300 hand tools (ESAW) |
| Sex | 1 | text | For instance 0= male 1= female |

### Example 5

Example 5 demonstrates via shown data element, relating to retirement for a relevant data base, entries that can relate to individual observables, but also that the data entries in the records can be made anonymous by the anonymizing interface. The actions and/or accidents apparent from the example 5 can be described according to a standard way, for example codes of European statistics on accidents at work (ESAW), or another suitable code. The lengths of the records can vary, or may be left empty in suitable part. The example 5 gives also one example on allotment of the system to allot an observable, with its status of presence in the system, to be used in for a data mining process. According to an embodiment, the anonymizing interface and/or the data mining means of the monitoring system can be arranged to collect the data entries form several data bases available for the monitoring system.

| # | Column | Length | Type | Notification |
|---|---|---|---|---|
| 1 | Retirement arrangement number | 15 | text | "12-3456789A" |
| 2 | Insurance taker | 35 | text | Name of the business |
| 3 | Number of employees | 6 | Numeric | 0 if not known |
| 4 | Code of the retirement type | 2 | text | "2", "0", "OK", "OY","2K", "9" |
| 5 | Clarification | 25 | text | According to a discretion below |
| 6 | Main class of diagnosis made | 3 | text | Main class within 3 codes, M07, T94 etc |
| 7 | Retirement date | 8 | date | YYYY.MM.DD (granting date) |
| 8 | Relevant year | 4 | Numeric | 2007 (sickness noticed, year) |
| 9 | Year of birth | 4 | Numeric | 1940 |
| 10 | Sex | 1 | text | M or F |
| 11 | Social security number | 11 | text | 010160-08XX |
| 12 | Compensation | 15 | numeric | Compensation in cents, for instance |

Discretion of the Clarification codes

| | |
|---|---|
| 2 | Partial disablement for working |
| 0 | Disablement for working |
| OK | Rehabilitation support |
| OY | Individual early retirement |
| 2K | Partial rehabilitation support |
| 9 | Rehabilitation fund |

### Example 6.

A non-restrictive example is shown. In a monitoring system according to an embodiment of the invention, a number of data bases are used for accessing to the observables as individual observables with their data items therein, as classified according to a standard way of classification for a search of employees that were injured in a working accident, for example. At the beginning, a search for the observables has been defined; a dynamic observable has been created. The dynamic observable would get the status that is defined via the individual observables found and scored according to a simple sum of the scores in each respective slot representing a data item for each observable. The scoring as such can be made by a known scoring algorithm as such. The search can be created as a totally new or as a modified one from a previous session. The system accesses to the data bases, uses the data base structures, and scores the data items in the allotment, according to a predefined scheme, but can also ask help for the user in an embodiment variant. Data items are selected that fit to the scoring pattern of the search. Suitable data items are scored according to the scoring pattern. This is performed until there are observables in the database selection available that fit to the scoring pattern, or all the material available that fit to the search criterion are dealt and/or scored.

The dynamic observable gets its status according to the scores in each slot of the status record, so that for each slot representing a feature of the dynamic observable, the scores are simply summed in this example in each slot for the corresponding observables. The dynamic observable can be provided with statistical shadow status that indicates the specifications of the data items, tolerances, variance etc. The shadow is thus as a measure of statistical reliability in a certain sense.

The allotment so made may need truncation or other processing, the scores can be normalized against the total score number for example, but not limiting only thereto. The status of the dynamic observable is set to the system status of the dynamic observable.

The system status of the dynamic observable is shown to the user, or is fed for a further processing. The user may want to review the search, scoring and/or data bases, and revise the search. The previous result may be saved for further retrieval. The user may so make several dynamic observables by combining the data items from various data bases, as based on the history data.

The monitoring system allows in an embodiment of the invention also finding trends in the history for the dynamic observable in respect of a scored feature. According to a variant of an embodiment the user is allowed to make assumptions on the features of the observables and so the dynamic observable can get a system status with predictive features that are based on the history information.

### Example 7.

According to an embodiment of the invention, a monitoring system according to an embodiment is used in a management system of a user for the user's needs, as a guidelines of decision making in the user's firm on the basis at least partly on the control data as made anonymous by an anonymous interface of the monitoring system. In an embodiment example, a user uses the control data on the estimating of the user's own state for a certain activity on the personnel and/or health aspects of the user via suitable dynamic observables, so that anonymous data on the dynamic observable is shown to the user via a presence server and/or via a server that is arranged to emulate a presence server's services .According to an embodiment the control data is sold as such, but according to an embodiment of the invention in a refined form or as a service.

Further examples and their implementation include:
A monitoring system for monitoring system status and/or change in the system status for an observable, in a predictive manner and/or the realization of said system status and/or change of said system status, The monitoring system is arranged to
- collect from a first monitoring system element, as arranged into a supervisors supervision, a first piece of information on said observable and/or
- collect from a second monitoring system element, as arranged into the supervisor's supervision, a second piece of information on said observable,
to be anonymously combined in the monitoring system for constituting a system status for said observable or changing a previous system status of said observable from a first system status to a second system status in a system status allotment comprising at least said first and second system status defined by the combined pieces of information, so that the system status of the observable is set to a system status value that belong to the system status allotment, for an observable made anonymous so that the monitoring system is arranged to be operable as a presence server system, for observing the status of said observable made anonymous by an anonymizing interface.

The monitoring system of the previous example wherein said observable comprises at least one ensemble of individual observables having a common feature in the information in the system status allotment.

The monitoring system of one of the previous examples wherein at least one of said first and second monitoring system elements is one in a plurality of monitoring system elements in the supervision of said supervisor.

The monitoring system of one of the previous examples wherein at least one of said first and second piece of information comprises an internal allotment for constituting a sub-allotment and/or a weight for said piece of information in said allotment and/or said sub-allotment.

The monitoring system of one of the previous examples wherein at least one of said first and second system elements comprises at least one of the following: a server, data base, a combination thereof.

The monitoring system of one of the previous examples wherein the system comprises allotment means arranged to constitute an allotment for said information.

The monitoring system of one of the previous examples wherein the system comprises weighting means arranged to give a weight for an individual piece of information in an item of said system allotment.

The monitoring system of one of the previous examples wherein the system comprises means for factor analysis for determination of the relevance of an individual pieces of information, for giving to an observable a status in the system status allotment via the factorization.

The monitoring system of the previous example wherein the system comprises predicting means arranged to predict a change of a system status of at least one ensemble of observables comprising at least one observable on the basis of said factor analysis.

The monitoring system of one of the previous examples wherein the system comprises reporting means arranged to report the system status of at least one ensemble of observables comprising at least one observable, to the supervisor.

The monitoring system of one of the previous examples wherein the system comprises reporting means arranged to report a prediction of the system status of at least one ensemble of observables comprising at least one observable to the supervisor.

The monitoring system of the two previous examples wherein the system comprises reporting means arranged to report to the supervisor a difference in the system status of at least one ensemble of observables comprising at least one observable, in comparison to the said prediction of the system status of the same.

The monitoring system of one of the previous examples wherein the system is comprised in a presence information server.

The monitoring system of the previous example wherein the presence information server comprises a server for mobile communications.

The monitoring system of one of the previous examples wherein the system comprises a data mining means arranged to acquire information from a system element mountable to the monitoring system as its system element.

The monitoring system of one of the previous examples wherein the system comprises an anonymizing interface to anonymize information for presentation after factor analysis at a presence service and/or adapt information obtained via data mining to a system allotment of the monitoring system for presentation of information items on an ensemble of observables on a presence server.

The monitoring system of one of the previous examples wherein the system is arranged to score a system status given by the monitoring system.

The monitoring system of one of the previous examples where the system is arranged to compare a first system status score to a second system status score given by the monitoring system.

The monitoring system of one of the previous examples wherein the system is arranged to advice at least one observable for actions aiming to changing its system status by an optimization criteria defined by a score difference of said first system status score and said second system status score given by the monitoring system.

The monitoring system of one of the previous examples wherein the observable comprises at least one of the following: a business, a service provider, an employee and an employer.

The monitoring system of one of the previous examples wherein the system comprises a presence server for handling system status of at least one individual observable and/or system status of at least one dynamic observable.

The monitoring system of one of the previous examples wherein the system comprises user interface, presence server emulator, a factor analysis means and/or data mining means combined to the interface.

A business method comprising use of said monitoring system wherein a score is associated to a pre-determined monetary value according to a pre-determined rule for a dynamic observable or its feature in the allotment.

A monitoring method of monitoring observables wherein the method comprises
- accessing to at least one data base,
- emulating and/or using a presence service for getting information on the observables to be monitored for a system status as presence status in the presence service,
- mining data/information on the observables for their system status for a dynamic observable, via an out come of at least one dynamic observable, from at least one data base,
- interfacing said out come into an allotment of the system status, for presentation of the system status with said dynamic observable,
- addressing a system status for at least one dynamic observable.

A software code on a machine readable medium, comprising means to install and/or execute a system according to any one of the previous claims.

An anonymizing interface, arranged to read confidential information from the records having an allotment, from the data storage of the information, to observe records in it and to recognize from said records identity information so that when said records used as input to said anonymizing interface, it is arranged to output said records or certain parts of such without the original identity in the allotment of the output information.

The anonymizing interface of the previous example arranged to replace the original identity in a record by a temporal identity information.

The anonymizing interface of the previous example arranged to remove the temporal identity after a predetermined time.

The anonymizing interface of one of the previous examples comprising a microprocessors, memory, and a user interface as configured to output, as based on said input information, statistical information provided without original identity information comprised identity,

The anonymizing interface of one of the previous examples arranged to operate in an information network there between its two network elements.

The anonymizing interface of one of the previous examples arranged to be updateable by update means that are arranged to update the criteria collection on defining the identities.

The anonymizing interface of one of the previous examples comprising data base means arranged to read and/or save anonymous statistical information with an allotment.

A method to convert information with a first allotment from a first data storage into statistical information with a second allotment in another data storage. The method comprises:
- reading, as an input, a piece of information concerning the health of an individual or other confidential information,
- recognizing the identity field from the allotment of the information for said individual as based on a criteria collection,
- writing into a data base said piece of information concerning the health of an individual or other confidential information, without the identity of said individual, as an output of an anonymizing interface for constituting and/or maintaining said data base.

Said method wherein said reading and/or writing operations are repeated for saving information of a data base by said anonymizing interface.

A business method comprising:
subscribing by a subscriber a subscription as a stimulus on anonymous statistical information to be collected from a data base and to be delivered to said subscriber, wherein as a first response, the service provider provides at least a part of said subscribed information as anonymous statistical information to said subscriber, and as a second response, provides an invoice, according to an invoicing criteria, to said subscriber, on the provided information, wherein said data base is constituted and/or maintained according to a method of claim 34.

The business method of the previous example wherein the stimulus and a response to it are arranged to be communicatable over an information network.

The business method of the previous example wherein the invoice is arranged to be payable via utilization of the same information network / network protocol as utilized for the subscription and/or delivery.

The business method of one of the previous examples wherein the invoicing criteria comprises at least one provision of the following:
- provision to classify the subscribed information, ,
- provision related to the processor time in the information collecting,
- provision bound to the number of records in a class of resulting information,
- provision bound to the data transfer time and/or number of data packages.

An arrangement for collecting and/or storing statistical information, **characterized** in that it comprises at least one anonymizing interface arranged to operate at an interface of a first system part and a second system part for anonymizing information on an observable.

The arrangement of the previous example wherein said system parts are parts of the service provider's system, said system parts are parts of the subscriber's system and/or other system's parts.

The arrangement of the first example wherein the arrangement is arranged to collect, from a plurality of monitoring system elements comprising at least one monitoring system element, as arranged into a supervisors supervision, at least one piece of information on said observable.

The arrangement of the previous example wherein said piece of information is collected to be anonymously combined in the monitoring system for constituting a system status for said observable or changing a previous system status of said observable from a first system status to a second system status in a system status allotment comprising at least said first and second system status defined by the combined pieces of information, so that the system status of the observable is set to a system status value that belong to the system status allotment, for an observable made anonymous so that the monitoring system is arranged to be operable as a presence server system, for observing the status of said observable made anonymous by an anonymizing interface.

A management system that comprises said anonymizing interface arranged to convert identity comprising information to statistical information as control data of the management system.

The management system of the previous example that comprises said monitoring system.

## Claims

**1.** A monitoring system, wherein
the monitoring system (131) is connected to one or more databases (101, 102, 103, 104) including data entries on individuals, data entries in each of the one or more connected databases having a defined allotment for data, and representing status for said individual in the connected database;
the monitoring system (131) is configured to query a user for creating a search with attributes that constitute a status for searching data entries on individuals that are of interest to the user from the one or more connected databases;
the monitoring system is configured to use data entries of a group of individuals resulting from the search to form, for each individual in the group of individuals, a first system status having a defined allotment for data, and representing status for said individual in the monitoring system;
the monitoring system is configured to expose the first system statuses to a statistical analysis to form a second system status representing status for the group of individuals in the monitoring system;
the monitoring system is configured to present the second system status to the user of the monitoring system.

**2.** A monitoring system according to a claim 1, **wherein** the monitoring system is configured to
score searched data entries according to a predefined scheme; and
use the scores of the data entries to form the second system status.

**3.** A monitoring system according to claim 2, **wherein** the monitoring system is configured to use a defined slot in the data entries of a first system status to represent a feature of the second system status, and to use scores of the data entries of that defined slot to form the second system status.

**4.** A monitoring system according to a claim 3, **wherein** the monitoring system is configured to sum the scores of the data entries of the defined slot to form the second system status.

**5.** A monitoring system according to claim 3 or 4, **wherein** the monitoring system is configured to save the second system status for further retrieval.

**6.** A monitoring system according to claim 5, **wherein** the monitoring system is configured to use the scores of the data entries of the defined slot in saved second system statuses to form a trend for the feature of the second system status.

**7.** A monitoring system according to any of the preceding claims 2 to 6, **wherein** the monitoring system is configured to anonymise the first system statuses of the group of individuals by converting information on an individual in each first system status into a form in which identity of the individual is not identifiable.

**8.** A monitoring system according to claim 7, **wherein** the monitoring system is configured to anonymise data in the first system statuses by removing original identities from the first system statuses.

**9.** A monitoring system according to a claim 8, **wherein** the monitoring system is configured to anonymise data in the first system statuses by giving to each first system status a temporal identity and to communicate the temporal identity also into a system of a database, which a data entry with the original identity came from.

**10.** A monitoring method, comprising
- accessing one or more databases including data entries on individuals, data entries in each of the one or more connected databases having a defined allotment for data, and representing status for said individual in the connected database;
- querying a user for creating a search with attributes that constitute a status for searching data entries on individuals that are of interest to the user from the one or more connected databases;
- using data entries of a group of individuals resulting from the search to form, for each individual in the group of individuals, a first system status having a defined allotment for data, and representing status for said individual in the monitoring system;
- exposing the first system statuses to a statistical analysis to form a second system status representing status for the group of individuals in the monitoring system;
- presenting the second system status to the user of the monitoring system.

**11.** A monitoring method according to a claim 10, further including
- scoring searched data entries according to a predefined scheme; and
- using the scores of the data entries to form the second system status.

**12.** A monitoring method according to claim 11, further including
- using a defined slot in the data entries of a first system status to represent a feature of the second system status, and
- using scores of the data entries of that defined slot to form the second system status.

**14.** A monitoring method according to claim 12 or 13, further including saving the second system statuses for further retrieval and using the scores of the data entries of the defined slot in saved second system statuses to form a trend for the feature of the second system status.

**15.** A computer program comprising program code means for performing the steps of the method of any of claims 10 to 14, when said program is run on a server computer of a monitoring system.
